# EUROPÄISCHE PATENTSCHRIFT

(11) **EP 2 163 230 B1**
(45) Veröffentlichungstag und Bekanntmachung des Hinweises auf die Patenterteilung: **27.03.2013**
(21) Anmeldenummer: 08016077.3
(22) Anmeldetag: 12.09.2008
(51) Int. Cl.: A61F 15/00, B65D 75/32, A61B 19/02, A61M 35/00

(54) **Packung für chirurgische Hilfsmittel, insbesondere für Präpariertupfer**
Packaging for surgical aids, in particular preparation swabs
Emballage pour dispositif d'aide chirurgical, en particulier pour brosse à pocher de préparation

(43) Veröffentlichungstag der Anmeldung: 17.03.2010
(73) Patentinhaber: NOBA Verbandmittel Danz GmbH u. Co. KG, 58300 Wetter-Wengern (DE)
(72) Erfinder: Danz, Sebastian, 58300 Wetter (DE)
(74) Vertreter: Rohmann, Michael

(56) Entgegenhaltungen:
- FR-A- 2 403 948
- US-A- 3 759 375
- US-A- 4 373 631
- US-A1- 2002 027 088

## Beschreibung

Die Erfindung betrifft eine Packung für Tupfer, insbesondere für Tupfer Präpariertupfer. Eine Mehrzahl derartiger Tupfer, insbesondere Präpariertupfer werden normalerweise bei Operationen am menschlichen Körper benötigt. Die Tupfer werden vor und nach der Operation genau abgezählt um zu gewährleisten, dass kein Tupfer nach der Operation im menschlichen Körper verbleibt.

Aus EP 0 401 744 B1 ist eine Packung der eingangs genannten Art bekannt. Diese Packung ist als Faltschachtel aus Karton ausgebildet und weist rechteckige Aufnahmen für die Tupfer auf, die durch Längsstege und Querstege aus Karton voneinander getrennt sind. Die Aufnahmen sind jeweils mit einem einzigen Tupfer befüllt. Die Größe der Aufnahmen soll so vorgesehen sein, dass jede der Aufnahmen gerade einen Tupfer, nicht aber zwei Tupfer aufnehmen kann. Dadurch soll erreicht werden, dass nach der Operation rücklaufende Tupfer wieder je für sich in eine der separaten Aufnahmen zurückgelegt werden. Hierdurch soll die Kontrolle über die Anzahl der vor und nach der Operation vorhandenen Tupfer erleichtert werden. Allerdings können sowohl die unbenutzten Tupfer als auch die benutzen Tupfer bei der Handhabung der Packung und insbesondere beim Öffnen oder Schließen der Packung leicht aus ihren Aufnahmen herausfallen, so dass hierdurch eine Fehleranfälligkeit bei der Kontrolle der Tupferanzahl bedingt wird.

Der Erfindung liegt das technische Problem zugrunde eine Packung der eingangs genannten Art anzugeben, die auf einfache Weise zu handhaben ist, bei der die Tupfer sicher und unverlierbar in den Aufnahmen gehalten werden und bei der die Tupfer nichtsdestoweniger nach ihrem Gebrauch einfach und problemlos in die Aufnahmen zurückführbar sind.

Zur Lösung dieses technischen Problems lehrt die Erfindung eine Packung für Tupfer, insbesondere für Präpariertupfer, wobei die Packung eine Mehrzahl von Aufnahmen für je ein Tupfer aufweist,
wobei die Größe der Aufnahmen mit der Maßgabe eingerichtet ist, dass in jeder Aufnahme lediglich ein Tupfer aufgenommen werden kann und wobei das Tupfer klemmend in einer Aufnahme gehalten wird.

In besonders vorteilhafter Ausgestaltung der Erfindung handelt es sich um einen Tupfer und sehr bevorzugt um einen Präpariertupfer. Ein Tupfer wird also in der zugeordneten Aufnahme gleichsam eingeklemmt und unter der Klemmwirkung unverlierbar in der Aufnahme gehalten. Nichtsdestoweniger ist die Aufnahme mit der Maßgabe eingerichtet, dass der Tupfer auf einfache Weise in die Aufnahme zurückgeführt bzw. zurückgedrückt werden kann. Nach besonders bevorzugter Ausführungsform der Erfindung weist dazu eine Aufnahme zumindest eine in Richtung des aufgenommenen Tupfers vorspringende Klemmnase für die klemmende Halterung des Tupfers auf. Zweckmäßigerweise sind zumindest zwei solcher Klemmnasen, bevorzugt zwei Klemmnasen vorgesehen. Es liegt dabei im Rahmen der Erfindung, dass die Klemmnasen an zwei gegenüberliegenden Seiten der Aufnahme angeordnet sind. In besonders vorteilhafter Ausgestaltung der Erfindung sind zwei Klemmnasen unmittelbar gegenüberliegend an zwei gegenüberliegenden Seiten der Aufnahme angeordnet, so dass sie zwischen sich einen Klemmspalt für das Tupfer bilden.

Gemäß einer Ausführungsform ist die Längserstreckung einer Aufnahme größer als ihre Quererstreckung und die zumindest eine Klemmnase ist dabei an einer Längsseite bzw. an einer der längeren Seiten der Aufnahme angeordnet. Zweckmäßigerweise ist die Klemmnase dabei in Bezug auf die Länge der Längsseite mittig bzw. in etwa mittig an der Längsseite angeordnet. Vorzugsweise sind zwei Klemmnasen an den beiden gegenüberliegenden Längsseiten bzw. längeren Seiten der Aufnahme vorgesehen und bevorzugt liegen beide Klemmnasen unmittelbar gegenüber, so dass sie zwischen sich einen Klemmspalt bilden. Empfohlenermaßen sind die beiden Klemmnasen dabei jeweils in Bezug auf die Länge der Längsseiten mittig bzw. in etwa mittig an einer Längsseite angeordnet. Gemäß einer Ausführungsvariante ist die Längserstreckung einer Aufnahme doppelt so groß bzw. etwa doppelt so groß wie ihre Quererstreckung oder zumindest doppelt so groß wie ihre Quererstreckung.

Zweckmäßigerweise erstreckt sich die zumindest eine Klemmnase über den größten Teil der Tiefe t einer Aufnahme. Größter Teil meint dabei insbesondere zumindest 50 % der Tiefe t, vorzugsweise zumindest 60 % der Tiefe t und bevorzugt zumindest 70 % der Tiefe t. Eine besonders bevorzugte Ausführungsform der Erfindung ist dadurch gekennzeichnet, dass die Aufnahmen an die Form der zugeordneten der zugeordneten Tupfer angepasst sind. Gemäß dieser bevorzugten Ausführungsform ist eine Aufnahme also nicht nur an die Größe sondern auch an die Form des zugeordneten Tupfers angepasst. Dass eine Aufnahme an die Form des zugeordneten Tupfer angepasst ist meint insbesondere, dass die Aufnahme mit Ausnahme des Bereichs der Klemmnasen an die Form des Tupfer angepasst ist. Wenn gemäß einer Ausführungsvariante die Längserstreckung des des Tupfers größer ist als seine Quererstreckung sind auch die Längserstreckung und die Quererstreckung der Aufnahme angepasst und die Längserstreckung der Aufnahme ist größer als die Quererstreckung der Aufnahme. Zweckmäßigerweise entspricht die Tiefe einer Aufnahme der Quererstreckung des Tupfers oder in etwa der Quererstreckung des chirurgischen Tupfers. Gemäß bevorzugter Ausführungsvariante ist eine Aufnahme im Querschnitt - parallel zur Fläche der Aufnahmeöffnung - hantelförmig bzw. in etwa hantelförmig ausgebildet. Die Engstelle des hantelförmigen Querschnitts entspricht dann dem Klemmspalt, der durch zwei gegenüberliegende Klemmnasen realisiert wird.

Empfohlenermaßen ist eine Aufnahme als Aufnahmemulde ausgebildet. Aufnahmemulde meint dabei insbesondere eine Aufnahme, die zumindest außerhalb des Bereichs der Klemmnasen ausschließlich abgerundete Wandungen bzw. sphärisch geformte Wandungen oder im Wesentlichen abgerundete Wandungen bzw. sphärisch geformte Wandungen aufweist. Es liegt im Rahmen der Erfindung, dass zumindest außerhalb des Bereichs der Klemmnasen keine scharfen Ecken bzw. Kanten oder quasi keine scharfen Ecken bzw. Kanten vorgesehen sind.

Eine besonders bevorzugte Ausführungsform der Erfindung ist dadurch gekennzeichnet, dass die erfindungsgemäße Packung ein tiefgezogenes Basisteil aus Kunststoff aufweist, in dem die Aufnahmen bzw. Aufnahmemulden ausgebildet sind. Die Aufnahmen bzw. Aufnahmemulden sind also Bestandteil des tiefgezogenen Basisteils aus Kunststoff. Zweckmäßigerweise handelt es sich um ein einstückiges tiefgezogenes Basisteil aus Kunststoff. Diese Ausführungsform hat sich besonders bewährt. Dabei kann die Klemmwirkung für die chirurgischen Hilfsmittel besonders einfach und funktionssicher realisiert werden.

Zweckmäßigerweise ist das Basisteil mit einer ebenen bzw. im Wesentlichen ebenen Oberfläche ausgestattet in welcher Oberfläche sich die Aufnahmeöffnungen der Aufnahmen bzw. Aufnahmemulden befinden. Ebene bzw. im Wesentlichen ebene Oberfläche des Basisteils meint hier also, dass die Oberfläche abgesehen von den darin vorgesehenen Aufnahmeöffnungen eben bzw. im Wesentlichen eben ausgebildet ist. In besonders vorteilhafter Ausgestaltung der Erfindung ist das Basisteil als Platte ausgebildet und die Aufnahmen bzw. Aufnahmemulden kragen aus der Unterseite dieser Platte vor. Auf der der Unterseite gegenüberliegenden Plattenseite ist zweckmäßigerweise die vorgenannte ebene bzw. im Wesentlichen ebene Oberfläche vorgesehen. Es liegt im Rahmen der Erfindung, dass auch die Unterseite der Platte - mit Ausnahme der vorkragenden Aufnahmen bzw. Aufnahmemulden - eben bzw. im Wesentlichen eben ausgebildet ist.

Es empfiehlt sich, dass die ebene bzw. im Wesentlichen ebene Oberfläche des Basisteils im verschlossenen Zustand der Packung von einer Verschlussfolie formschlüssig abgedeckt wird. Es liegt dabei im Rahmen der Erfindung, dass die Verschlussfolie aus Kunststoff besteht. Formschlüssig abgedeckt meint hier insbesondere, dass die Verschlussfolie unmittelbar auf der ebenen bzw. im Wesentlichen ebenen Oberfläche des Basisteils aufliegt, so dass alle Aufnahmen bzw. Aufnahmeöffnungen der Aufnahmen von der Verschlussfolie verschlossen werden. Zweckmäßigerweise ist die Verschlussfolie im verschlossenen Zustand der Packung an den Rändern der Packung luftdicht mit dem Basisteil verbunden. Dabei ist die Verschlussfolie an den Rändern der Packung vorzugsweise luftdicht mit dem Basisteil verklebt und/oder verschweißt. Es liegt im Rahmen der Erfindung, dass die Verschlussfolie in den übrigen Bereichen des Basisteils ohne Fixierung bzw. lose auf dem Basisteil bzw. auf der Oberfläche des Basisteils aufliegt. Es liegt fernerhin im Rahmen der Erfindung, dass die Verschlussfolie zum Öffnen der Packung von dem Basisteil abziehbar ist. Zweckmäßigerweise wird bei diesem Abziehen der Verschlussfolie eine Klebeverbindung bzw. Adhäsivverbindung zwischen Verschlussfolie und Basisteil gelöst. Nach empfohlener Ausführungsform weist die Verschlussfolie einen Manipulierstreifen auf, mit dessen Hilfe die Verschlussfolie von dem Basisteil abziehbar ist. Der Manipulierstreifen ist dabei zweckmäßigerweise nicht an dem Basisteil fixiert sondern liegt gleichsam lose auf dem Basisteil auf.

Gemäß bevorzugter Ausführungsvariante ist zumindest ein Teil der Packung transparent ausgebildet. Vorzugsweise ist zumindest die Verschlussfolie transparent ausgebildet und bevorzugt ist sowohl das Basisteil als auch die Verschlussfolie transparent ausgebildet. - Zweckmäßigerweise sind die Aufnahmen bzw. Aufnahmemulden in dem Basisteil in parallel zueinander angeordneten Reihen vorgesehen.

Der Erfindung liegt zunächst die Erkenntnis zugrunde, dass die erfindungsgemäße Packung auf einfache und problemlose Weise handhabbar ist. Die Tupfer werden insbesondere unmittelbar vor ihrem Gebrauch und vor allem beim Öffnen der Packung fest und unverlierbar in den Aufnahmen gehalten, so dass ein unerwünschtes Herausfallen aus der Packung effektiv vermieden werden kann. Nichtsdestoweniger können gebrauchteTupfer auf einfache und zwanglose Weise wieder in die Aufnahmen zurückgeführt werden und dort werden die Tupfer dann wiederum in vorteilhafter Weise fest und unverlierbar gehalten. Es ist weiterhin darauf hinzuweisen, dass die erfindungsgemäße Packung auf einfache, wenig aufwendige und insbesondere kostengünstige Weise herstellbar ist.

Nachfolgend wird die Erfindung anhand einer lediglich ein Ausführungsbeispiel darstellenden Zeichnung näher erläutert. Es zeigen in schematischer Darstellung:
- **Fig. 1**: eine perspektivische Ansicht einer erfindungsgemäßen Packung,

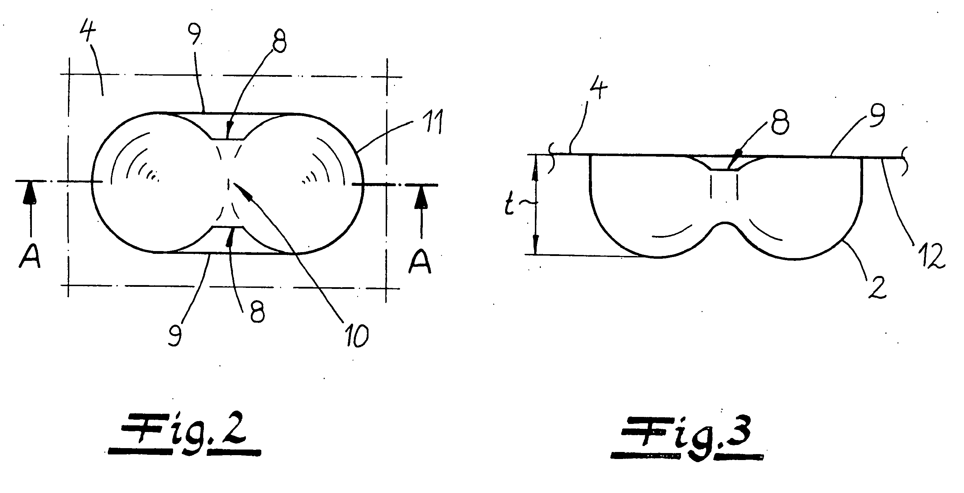

- **Fig. 2**: eine Draufsicht auf eine Aufnahme der erfindungsgemäßen Packung und
- **Fig. 3**: eine Seitenansicht einer Aufnahme im Schnitt A-A.

Die Figuren zeigen eine Packung für Tupfer, bei denen es sich im Ausführungsbeispiel um Präpariertupfer 1 handelt. Die Packung weist eine Mehrzahl von als Aufnahmemulden 2 ausgebildeten Aufnahmen für je einen Präpariertupfer 1 auf. Die Größe der Aufnahmemulden 2 ist mit der Maßgabe eingerichtet, dass in jeder Aufnahmemulde 2 lediglich ein Präpariertupfer 1 aufgenommen werden kann. Im Übrigen ist die Form der Aufnahmemulden 2 an die Form der Präpariertupfer 1 angepasst.

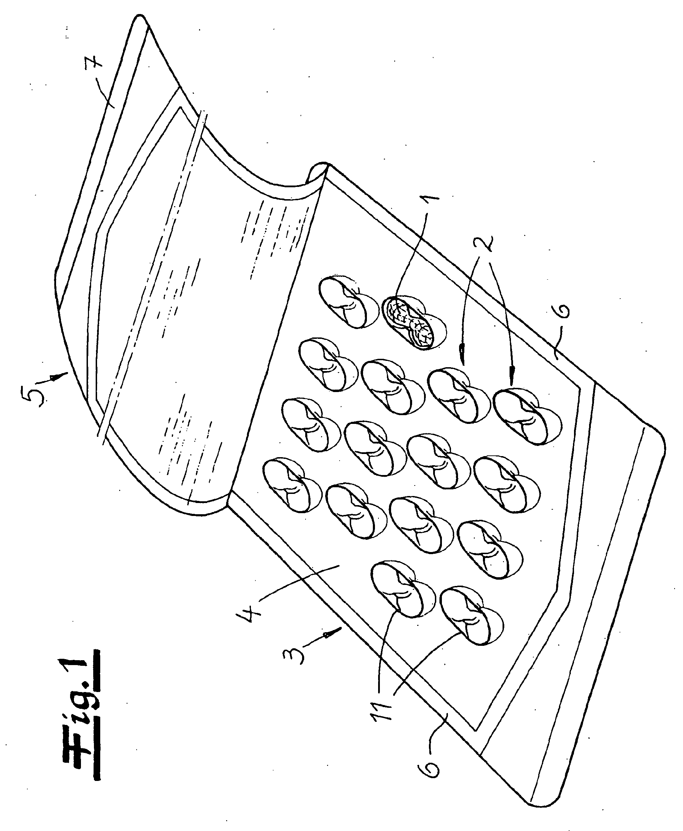

Vorzugsweise und im Ausführungsbeispiel weist die Packung ein einstückiges tiefgezogenes Basisteil 3 aus Kunststoff auf, in dem die Aufnahmemulden 2 vorgesehen sind. Das Basisteil 3 hat bevorzugt eine ebene Oberfläche 4, in der sich die Aufnahmeöffnungen 11 der Aufnahmemulden 2 befinden. Empfohlenermaßen und im Ausführungsbeispiel ist das Basisteil 3 als Platte ausgebildet und die Aufnahmemulden 2 kragen aus der Unterseite 12 dieser Platte vor. Die ebene Oberfläche 4 des Basisteils 3 wird im verschlossenen Zustand der Packung von einer Verschlussfolie formschlüssig abgedeckt. Die vorzugsweise aus Kunststoff bestehende Verschlussfolie 5 ist im verschlossenen Zustand der Packung an den Rändern 6 der Packung luftdicht mit dem Basisteil 3 verbunden, insbesondere luftdicht mit dem Basisteil 3 über eine Adhäsivverbindung verbunden. Die Verschlussfolie 5 ist zum Öffnen der Packung von dem Basisteil 3 abziehbar. Fig. 1 zeigt den geöffneten Zustand der Verschlussfolie 5. Die Verschlussfolie 5 weist vorzugsweise und im Ausführungsbeispiel einen Manipulierstreifen 7 auf, mit dessen Hilfe die Verschlussfolie 5 von dem Basisteil 3 abziehbar ist. Zweckmäßigerweise liegt der Manipulierstreifen 7 in verschlossenem Zustand der Packung lose und ohne Verbindung mit dem Basisteil 3 auf der Oberfläche 4 des Basisteils 3 auf. Im Ausführungsbeispiel nach Fig. 1 sind vier Reihen von jeweils vier Aufnahmemulden 2 vorgesehen. Die Reihen sind parallel zueinander angeordnet.

Erfindungsgemäß werden die Präpariertupfer 1 jeweils klemmend in einer Aufnahmemulde 2 gehalten. Dazu weist im Ausführungsbeispiel eine Aufnahmemulde 2 zwei gegenüberliegende in Richtung des aufgenommenen Präpariertupfers 1 vorspringende Klemmnasen 8 für die klemmende Halterung des Präpariertupfers 1 auf. Im Ausführungsbeispiel ist die Längserstreckung einer Aufnahmemulde 2 größer als ihre Quererstreckung und die beiden Klemmnasen sind an den beiden gegenüberliegenden Längsseiten einer Aufnahmemulde 2 angeordnet. Vorzugsweise und im Ausführungsbeispiel ist jede Klemmnase 8 dabei in Bezug auf die Länge einer Längsseite 9 mittig an dieser Längsseite 9 angeordnet. Zweckmäßigerweise und im Ausführungsbeispiel erstreckt sich eine Klemmnase 8 über den größten Teil der Tiefe t einer Aufnahmemulde 2. Die gegenüberliegenden Klemmnasen 8 definieren einen Klemmspalt 10 für die klemmende Halterung eines Präpariertupfers 1. Aufgrund dieses Klemmspaltes 10 ist eine Aufnahmemulde 2 im Querschnitt hantelförmig ausgebildet.

## Patentansprüche

1. Packung für Tupfer, insbesondere für Präpariertupfer, wobei die Packung eine Mehrzahl von Aufnahmen für je einen Tupfer aufweist, wobei die Größe der Aufnahmen mit der Maßgabe eingerichtet ist, dass in jeder Aufnahme lediglich ein Tupfer aufgenommen werden kann, wobei die Aufnahmen an die Form der zugeordneten Tupfer angepasst sind und wobei ein Tupfer klemmend in eine Aufnahme gehalten wird.

2. Packung nach Anspruch 1, wobei eine Aufnahme zumindest eine in Richtung des aufgenommenen Tupfers vorspringende Klemmnase (8) für die klemmende Halterung des chirurgischen Hilfsmittels aufweist.

3. Packung nach Anspruch 2, wobei die Längserstreckung einer Aufnahme größer ist als ihre Quererstreckung und wobei die zumindest eine Klemmnase (8) an einer Längsseite der Aufnahme angeordnet ist.

4. Packung nach einem der Ansprüche 2 oder 3, wobei sich eine Klemmnase (8) über den größten Teil der Tiefe t einer Aufnahme erstreckt.

5. Packung nach einem der Ansprüche 1 bis 4, wobei eine Aufnahme als Aufnahmemulde ausgebildet ist.

6. Packung nach einem der Ansprüche 1 bis 5, wobei die Packung ein tiefgezogenes Basisteil (3) aus Kunststoff aufweist, in dem die Aufnahmen bzw. Aufnahmemulden (2) vorgesehen sind.

7. Packung nach einem der Ansprüche 1 bis 6, wobei ein Basisteil (3) mit einer ebenen bzw. im Wesentlichen ebenen Oberfläche (4) vorgesehen ist, in welcher Oberfläche (4) sich die Aufnahmeöffnungen (11) der Aufnahmen bzw. Aufnahmemulden (2) befinden.

8. Packung nach einem der Ansprüche 1 bis 7, wobei ein Basisteil (3) in Form einer Platte vorgesehen ist und wobei die Aufnahmen bzw. Aufnahmemulden (2) aus der Unterseite (12) der Platte vorkragen.

9. Packung nach Anspruch 7, wobei die ebene bzw. im Wesentlichen ebene Oberfläche (4) des Basisteils (3) im verschlossenen Zustand der Packung von einer Verschlussfolie (5) formschlüssig abgedeckt wird.

10. Packung nach Anspruch 9, wobei die Verschlussfolie (5) im verschlossenen Zustand der Packung an den Rändern (6) der Packung luftdicht mit dem Basisteil (3) verbunden ist.

11. Packung nach einem der Ansprüche 9 oder 10, wobei die Verschlussfolie (5) zum Öffnen der Packung von dem Basisteil (3) abziehbar ist.

12. Packung nach einem der Ansprüche 9 bis 11, wobei die Verschlussfolie (5) einen Manipulierstreifen (7) aufweist, mit dessen Hilfe die Verschlussfolie (5) von dem Basisteil (3) abziehbar ist.

13. Packung nach einem der Ansprüche 1 bis 12, wobei zumindest ein Teil der Packung transparent ausgebildet ist.

14. Packung nach Anspruch 13, wobei das Basisteil (3) und/oder die Verschlussfolie (5) transparent ausgebildet ist/sind.

## Claims

1. A package for swabs, in particular for preparation swabs, wherein the package comprises a multitude of receiving devices each for one swab, wherein the size of the receiving devices is such that in each receiving device only one swab can be received, wherein the receiving devices match the shape of the associated swabs, and wherein a swab is held in a clamped manner in a receiving device.

2. The package according to claim 1, wherein a receiving device comprises at least one clamping lug (8), which projects in the direction of the received swab, for holding the swab in a clamped manner.

3. The package according to claim 2, wherein the longitudinal extension of a receiving device is greater than its crosswise extension, and wherein the at least one clamping lug (8) is arranged on a longitudinal side of the receiving device.

4. The package according to one of claims 2 or 3, wherein a clamping lug (8) extends over the largest part of the depth t of a receiving device.

5. The package according to any one of claims 1 to 4, wherein a receiving device is designed as a receiving depression.

6. The package according to any one of claims 1 to 5, wherein the package comprises a deep-drawn base part (3) made of plastic, in which base part (3) the receiving devices or receiving depressions (2) are provided.

7. The package according to any one of claims 1 to 6, wherein a base part (3) with a flat or essentially flat surface (4) is provided, in which surface (4) the receiving openings (11) of the receiving devices or receiving depressions (2) are arranged.

8. The package according to any one of claims 1 to 7, wherein a base part (3) in the form of a plate is provided, and wherein the receiving devices or receiving depressions (2) project from the underside (12) of the plate.

9. The package according to claim 7, wherein the flat or essentially flat surface (4) of the base part (3) in the closed state of the package is covered in a positive-locking manner by a sealing foil (5).

10. The package according to claim 9, wherein the sealing foil (5) in the closed state of the package on the margins (6) of the package is connected in an airtight manner with the base part (3).

11. The package according to one of claims 9 or 10, wherein for opening the package the sealing foil (5) can be pulled from the base part (3).

12. The package according to any one of claims 9 to 11, wherein the sealing foil (5) comprises a manipulation strip (7) by means of which the sealing foil (5) can be pulled from the base part (3).

13. The package according to any one of claims 1 to 12, wherein at least part of the package is designed so as to be transparent.

14. The package according to claim 13, wherein the base part (3) and/or the sealing foil (5) are/is designed so as to be transparent.

## Revendications

1. Emballage pour tampons, notamment pour tampons de préparation, l'emballage comportant une pluralité de logements pour chaque fois un tampon, la dimension des logements étant ajustée avec le critère que dans chaque logement, un seul tampon peut être réceptionné, les logements étant adaptés à la forme des tampons associés et un tampon étant maintenu par serrage dans un logement.

2. Emballage selon la revendication 1, un logement comportant au moins un talon de serrage (8) saillant en direction du tampon réceptionné pour la fixation par serrage de l'auxiliaire chirurgical.

3. Emballage selon la revendication 2, l'extension longitudinale d'un logement étant supérieure à son extension transversale et l'au moins un talon de serrage (8) étant disposé sur un côté longitudinal du logement.

4. Emballage selon l'une quelconque des revendications 2 ou 3, un talon de serrage (8) s'étendant sur la majeure partie de la profondeur t d'un logement.

5. Emballage selon l'une quelconque des revendications 1 à 4, un logement étant conçu en tant que cavité de logement.

6. Emballage selon l'une quelconque des revendications 1 à 5, l'emballage comportant une partie de base (3) en matière plastique emboutie, dans laquelle sont prévu(e)s les logements ou les cavités de logement (2).

7. Emballage selon l'une quelconque des revendications 1 à 6, une partie de base (3) avec une surface (4) plane ou sensiblement plane étant prévue, dans laquelle surface (4) se trouvent les ouvertures de logement (11) des logements ou cavités de logement (2).

8. Emballage selon l'une quelconque des revendications 1 à 7, une partie de base (3) en forme d'une plaque étant prévue et les logements ou cavités de logement (2) saillant hors de la face inférieure (12) de la plaque.

9. Emballage selon la revendication 7, à l'état fermé de l'emballage, la surface (4) plane ou sensiblement plane de la partie de base (3) étant recouverte par complémentarité de forme par un film de fermeture (5).

10. Emballage selon la revendication 9, à l'état fermé de l'emballage, sur les bords (6) de l'emballage, le film de fermeture (5) étant relié de manière étanche à l'air avec la partie de base (3).

11. Emballage selon l'une quelconque des revendications 9 ou 10, pour ouvrir l'emballage, le film de fermeture (5) étant pelable de la partie de base (3).

12. Emballage selon l'une quelconque des revendications 9 à 11, le film de fermeture (5) comportant un ruban de manipulation (7) à l'aide duquel le film de fermeture (5) est pelable de la partie de base (3).

13. Emballage selon l'une quelconque des revendications 1 à 12, au moins une partie de l'emballage étant conçue en version transparente.

14. Emballage selon la revendication 13, la partie de base (3) et/ou le film de fermeture (5) étant conçu(e/s) en version transparente.
